Europäisches Patentamt

European Patent Office

Office européen des brevets

(11) Publication number: **0 068 701**
A2

## (12) EUROPEAN PATENT APPLICATION

(21) Application number: 82303071.3

(22) Date of filing: 14.06.82

(51) Int. Cl.³: **C 12 N 15/00**, C 12 P 21/02, C 07 C 103/52, C 07 H 21/04

(30) Priority: 19.06.81 US 275161

(43) Date of publication of application: 05.01.83 Bulletin 83/1

(84) Designated Contracting States: **BE CH DE FR GB IT LI NL SE**

(71) Applicant: **Canadian Patents and Development Limited, 275 Slater Street, Ottawa Ontario, K1A OR3 (CA)**

(72) Inventor: **Narang, Saran A., 30 Higgins Road, Ottawa Ontario (CA)**
Inventor: **Wu, Ray J., 111 Christopher Circle, Ithaca N.Y. 14850 (US)**

(74) Representative: **Lambert, Hugh Richmond et al, D. YOUNG & CO. 10 Staple Inn, London, WC1V 7RD (GB)**

(54) Proinsulin genes with modified C-chain.

(57) Human-like proinsulin gene analogs have been synthesized by a combination of chemical and enzymatic methods. A number of different human-like proinsulin gene analogs with altered C-chains have been designed and can be readily constructed as described. As a part of the strategy, an adaptor for trimming DNA has been used to recover the A-chain insulin gene with the desired sequence from a hybrid plasmid; a related adaptor for trimming DNA has been used to shorten the C-chain gene. The synthetic proinsulin gene has been joined to a replicable cloning vehicle and the hybrid DNA transferred to a host cell. The transformed host cells can be grown under selected conditions to yield the proinsulin analogs.

PROINSULIN GENES WITH MODIFIED C-CHAIN    0068701

Field of the Invention

This invention is concerned with the synthesis of exact DNA genetic sequences and the joining of the synthetic gene to replicable cloning vehicles. Of particular concern is the total synthesis of the human-like proinsulin gene analogs, the insertion of the gene analogs into cloning vehicles, and the transferring of the hybrid DNA molecules into host cells, the transformed cells thus having the ability to produce the specified human-like proinsulin protein. The use of several adaptors for trimming DNA has been described, in particular trimming C-chain DNA down to a desired length.

Background and Prior Art

In recent years, methods have been developed (see "Molecular Cloning of Recombinant DNA", eds., W.A. Scott and R. Werner, Academic Press Inc., 1977), (1) for the _in vitro_ joining by DNA ligase of a DNA segment to be cloned to a cloning vehicle (DNA capable of independent replication),

(2) for introducing the hybrid DNA molecule (recombinant DNA) into a suitable host·cell,

(3) for selecting and identifying the transformed cells carrying the desired hybrid DNA (cloned DNA as a hybrid DNA),

(4) for amplifying the desired cloned DNA in the transformed cells, and

(5) for expressing the cloned DNA as a protein product. In most reported cases, DNA molecules isolated from cells or viruses have been fragmented by restriction enzyme digestion or by physical shearing or by reverse transcription copy of messenger to cDNA before cloning.

Protein synthesis in bacteria using a segment of transferred DNA derived from mouse as the blue print was shown by Chang et al. (Cell 6, 231-244, 1975). Still other examples of the cloning of natural foreign DNA have been described recently (Goeddel et al, Nature 281, 544-548, 1979; etc.).

Methods for the total chemical synthesis of oligodeoxynucleotides of up to 20-nucleotides-long have been well established by using either the phosphodiester method (H.G. Khorana, J. Mol. Biol. 72, 209, 1972) or the improved phosphotriester method (H.M. Hsiung and S.A. Narang, Nucleic Acids Res. <u>6</u>, 1371, 1979; S.A. Narang et al, Methods in Enzymology, Vol. <u>65</u>, 610, 1980, and Vol. <u>68</u>, 90, 1979). The latter method is now the preferred method because of its higher speed, better yield and purity of products, and has been used to prepare defined DNA sequences of longer length.

A few chemically-synthesized DNA sequences, such as the lactose operator (Marians, Wu, et al, Nature <u>263</u>, 744, 1976) and the tyrosine tRNA gene (Khorana, Science <u>203</u>, 614, 1979), have been successfully cloned in <u>E</u>. <u>coli</u> and the expression of the cloned DNA detected in subsequent cultures. Recent reports have indicated that human brain hormone somatostatin (Itakura et al, Science <u>198</u>, 1056, 1977) and human growth hormone (Goeddel et al, Nature <u>281</u>, 544, 1979) have been produced in a transformed bacterial host which had the transferred synthesized gene.

In the pancreas of animals, preproinsulin (S.J. Chan and D.F. Steiner, Proc. Nat. Acad. Sci. <u>73</u>, 1964, 1976) is synthesized as the precursor of insulin. The general structure of proinsulin is $NH_2$–B chain-(C chain)-A chain-COOH; it is converted to insulin by the action of peptidases in the pancreatic islet tissue which remove the C-chain by cleavage at the positions of the two arrows shown in Formula 1 for the human proinsulin (Oyer et al, J. Biol. Chem., <u>246</u>, 1375, 1971). The B-chain and A-chain of insulin are held together by two disulfide cross-linkages which are formed at the correct location at the stage of the proinsulin.

Using a biological method, Ullrich et al, (Science <u>196</u>, 1313, 1977) and Villa-Komoroff et al, (Proc. Nat. Acad. Sci. <u>75</u>, 3727, 1978) succeeded in cloning the coding region of rat proinsulin I. Using a chemical method, Crea et al, (Proc. Nat. Acad. Sci. <u>75</u>, 5765, 1978), synthesized, and Goeddel et al (Proc. Nat. Acad. Sci. <u>76</u>, 106, 1979), cloned, an insulin A-chain gene and a B-chain gene, separately. The codons selected for these synthetic genes were arbitrary and quite different from the natural human DNA sequence. On culturing, the bacteria produced an insulin A-chain protein and B-chain protein which were separately treated to remove the extraneous β-galactosidase and methionine.

In U.S. Patent Application Serial No. 843,422, filed Oct. 19, 1977, by R. Wu et al, and U.S. Patent Application Serial No. 129,880,

Formula 1   THE STRUCTURE OF HUMAN PROINSULIN.  B chain, amino acids 1-30, C peptide, amino acids 31-65; A chain, amino acids 66-86.  Residues 31, 32, 64, 65 will be excised during processing.

N-terminal end

1 Phe
|
Val
|                    5                          10                          15                    20      22
Asn-Glu-His-Leu-Cys-Gly-Ser-His-Leu-Val-Glu-Ala-Leu-Tyr-Leu-Val-Cys-Gly-Glu-Arg-Gly-Phe-Phe 25
                    |                                                                    \        Tyr
                    S                                                                     S       |
                    |                       A CHAIN                                       |       Thr
       70           S                          75                                         S       |
Val-Glu-Gln-Cys-Cys-Thr-Ser-Ile-Cys-Ser-Leu-Tyr-Glu-Leu-Glu-Asn-Tyr-Cys-Asn-COOH                Pro
       |     |_____|                            80              85                  |
       Ile        S     S                                              C-terminal end             Lys
       |                                                                                           |
       Gly                                                                                         Thr 30
       |    ←                                                                                  ←
65 Arg                                                                                            Arg
       |                                                                                           Arg
       Lys                                                                                         |
       |                                                                                           Glu
       Gln                                                                                         |
       |                                                                                           Ala
       Leu                                                                                         |
       |                                                                                           Glu 35
       Ser                                                                                         |
       /                                                                                           Asp
      /                           C PEPTIDE                                                         |
Gly-Glu-Leu-Ala-Leu-Pro-Gln-Leu-Ser-Gly-Ala-Gly-Pro-Gly-Gly-Gly-Leu-Glu-Val-Gln-Gly-Val-Gln-Leu
60                    55                    50                    45                    40

filed March 27, 1980, by S.A. Narang et al, synthetic adaptor molecules were described for attachment to the ends of DNA sequences, such as synthetic insulin A-chain and B-chain gene, for joining to cloning vehicles or other DNA. These adaptors comprised DNA (oligonucleotide) sequences having particular nucleotide segments which are recognition sites for restriction endonucleases and codon triplets. These adaptors can also be used to provide an enzyme recognition site on a duplex DNA sequence or to change from one type of site to another.

Summary of the Invention

Novel codon sequences for insulin C-chain gene (DNA) have been provided. Shorter or mini-C-chains have been developed and used to form analogs of proinsulin. A restriction enzyme recognition site was incorporated in these C-chains by selection of appropriate codons at a selected location to facilitate opening and insertion of a DNA information sequence.

The invention includes combining selected A-, B- and C-chains into novel proinsulin genes, ligating these genes to replicable cloning vehicles and transferring the resulting hybrid into host cells. In particular, for example, a synthetic human-like proinsulin gene (formula 2) has been synthesized by a combination of chemical and enzymatic methods. A start signal (ATG) and an EcoRI cohesive end (5' AATT) was placed at the 5' end (left-hand end) of the gene, and a stop signal (TGA) and a BamHI cohesive end (5' GATC) was placed at the 3' end of the gene. This adapted proinsulin gene was joined to a replicable cloning vehicle that can enter a cell and replicate itself together with the cloning vehicle. The transformed host cell progeny was shown by DNA sequence analysis to contain the exact input proinsulin gene. A plasmid cloning vehicle has been constructed which includes a lactose promoter and produces several hundred copies of the proinsulin gene per cell.

Formula 2 DNA SEQUENCE OF HUMAN-LIKE PROINSULIN GENE. The duplex structure represented by base pairs 1 through 258 constitutes the proinsulin gene. The B-chain, C-chain and A-chain start from base pairs 1, 91 and 196, respectively.

```
     EcoRI site   | B-chain
     ---- B-18----|       B-1                    B-2                   B-3
5'  AATTC CGG ATG| TTT GTC AAT CAG  CAC CTT TGT GGT  TCT CAC CTG GTG
                  | 1                                                    31

3'     G GCC TAC  AAA CAG TTA GTC GTG GAA  ACA CCA AGA GTG  GAC CAC
          B-19    |          B-10                B-11                 B-
```

```
         B-4                    B-5              B-6               B-7
5'  GAG GCT CTG TAC  CTG GTG TGT GGG GAA CGT GGT TTC  TTC TAC ACA CCC
                                           61                          83

3'  CTC CGA  GAC ATG GAC CAC  ACA CCC CTT GCA  CCA AAG AAG ATG  TGT GGG
      12         B-13              B-14             B-15              B-
```

```
                | C-chain
       B-8      |          C-1              C-2              C-3
5'  AAG ACC| CGT CGT  GAA GCT GAA GAC  CTT CAA GTG GGT  CAA GTT GAA CTT
          | 91                                   121

3'  TTC TGG| GCA GCA CTT CGA  CTT CTG GAA GTT CAC CCA GTT CAA  CTT GAA
      16    |       C-9              C-10             C-11             C-
```

```
       C-4              C-5              C-6                 C-7
5'  GGT GGG GGT  CCT GGT GCG GGT  TCT CTT CAA CCT  TTG GCT CTC GAG GGA

3'  CCA CGC CCA GGA CCA CGC  CCA AGA GAA  GTT GGA AAC CGA  GAG CTC CCT
      12        C-13              C-14             C-15              C-16
```

```
                        | A-chain
             C-8        |
5'  TCA CTT CAA AAG CGT| GGC ATT GTG GAG CAG TGC TGC ACC AGC ATC TGC
      181               | 196

3'  AGT GAA GTT TTC GCA| CCG TAA CAC CTC GTC ACG ACG TGG TCG TAG ACG
            C-17        |
```

```
5'  TCC CTC TAC CAA CTG GAG AAC TAC TGC AAC TGA G           3'
                      241                     258

3'  AGG GAG ATG GTT GAC CTC TTG ATG ACG TTG ACT CCTAG       5'
                                                BamHI site
```

Detailed Description and Preferred Embodiments

A. The Adaptors for Trimming DNA

The principle behind this type of retrieving (trimming) adaptor is based on the knowledge that certain restriction endonucleases such as HphI and MboII cleave DNA eight nucleotides away from their recognition sequence (R.J. Roberts, Methods in Enzymol. 68, 27, 1980). In formula 3, the five nucleotides with a line drawn above constitute the recognition sequence, where N stands for any nucleotide.

Formula 3 — Recognition sequence and mode of cleavage of HphI and MboII

(a) HphI    5'  $\overline{\text{G G T G A}}$ N N N N N N N N$^\downarrow$
            3'  C C A C T N N N N N N N$_\uparrow$


(b) MboII   5'  $\overline{\text{G A A G A}}$ N N N N N N N N$^\downarrow$
            3'  C T T C T N N N N N N N$_\uparrow$


We have designed and synthesized adaptors for trimming DNA as symmetric adaptors (non-symmetric adaptors based on formula 3 can also be used):

Formula 4 — HphI and MboII adaptors for trimming DNA

(a-1) HphI   5'  T C A C C $\overline{\text{G G T G A}}$
             3'  $\underline{\text{A G T G G}}$ C C A C T


(b-1) MboII  5'  T C T T C $\overline{\text{G A A G A}}$
             3'  $\underline{\text{A G A A G}}$ C T T C T


This type of adaptor for trimming DNA can be blunt-end ligated to any DNA from which 8 nucleotides at the end can be removed. MboII adaptor is used as an example to remove eight nucleotides N N N N N N N N from the end of a cloned insulin A-chain gene.

Formula 5 - Scheme for removing 8 base pairs from any DNA molecule using MboII retrieving adaptor

(b-2)

```
                                1                 8
5'  T C T T C G̅ ̅A̅ ̅A̅ ̅G̅ ̅A̅  N N N N N N N N  ┌────────┐  ─ ─ ─ ─ ─
                                                │ cloned │
                         +                      │ A-chain│
3'  A̲ ̲G̲ ̲A̲ ̲A̲ ̲G̲  C T T C T   N N N N N N N N  │ gene   │  ─ ─ ─ ─ ─
                                                └────────┘
```

                    │         blunt-end ligation (T₄ DNA ligase)
                    ▼

```
                            1                 8
5'  T C T T C G̅ ̅A̅ ̅A̅ ̅G̅ ̅A N N N N N N N N  ┌────────┐  ─ ─ ─ ─ ─
                                            │ cloned │
                                            │ A-chain│
3'  A̲ ̲G̲ ̲A̲ ̲A̲ ̲G̲ C T T C T N N N N N N N N  │ gene   │  ─ ─ ─ ─ ─
                                            └────────┘
```

                    │         MboII endonuclease
                    ▼

```
5'  T C T T C G̅ ̅A̅ ̅A̅ ̅G̅ ̅A N N N N N N N N  ┌────────┐  ─ ─ ─ ─
                                            │ cloned │
                              +             │ A-chain│
3'  A̲ ̲G̲ ̲A̲ ̲A̲ ̲G̲ C T T C T N N N N N N    'N' │ gene   │  ─ ─ ─ ─
                                            └────────┘
```

                    │         E. coli polymerase I + dXTP
                    ▼

```
      5'  ┌────────┐  ─ ─ ─ ─ ─
          │ cloned │
          │ A-chain│            +'N'
      3'  │ gene   │  ─ ─ ─ ─ ─
          └────────┘
```

The dXTP is chosen so that it is identical to the one adjacent to 'N' at the 3' end of the cloned A-chain gene, e.g. if the nucleotide "X" is C then dCTP is used.

In principle, adaptors for trimming DNA of this type containing 1 to 7 additional nucleotides beyond the MboII or HphI recognition sites can be used to trim off 7 to 1 base pairs from the ends of any DNA duplex (Narang et al, Nucleic Acids Res. Symposium Series No. 7, 377, 1980). This technique was used in modifying the insulin C-chain.

B. Chemical synthesis of deoxyribooligonucleotide fragments constituting the sequence of human proinsulin gene

The chemical synthesis of all the deoxyribooligonucleotides constituting the sequence of human proinsulin DNA as in formula 2 and various adaptors were achieved by the modified phosphotriester approach developed in one of our labs in 1973 (Can. J. Chem. 51, 3649, 1973; S. Narang et al, Methods of Enzymology 65, 610, 1980; and S. Narang et al,

Methods in Enzymology <u>68</u>, 90, 1979). The main features of our approach are:

(i) use of 5'-dimethoxytrityl-deoxyribomononucleoside 3'-phosphotriester (monomer) as a starting material;

(ii) condensation between 5'-dimethoxytrityl-deoxyribomononucleoside 3'-phosphodiester with a 5'-hydroxy containing component to yield a di-nucleotide containing an internucleotidic phosphotriester linkage as a neutral species which is amenable to all the conventional techniques of organic chemistry for their isolation; and

(iii) formation of the desired product in a higher yield because of the absence of side products.

The synthesis of all the deoxyribooligomers (10-20 units length) were constructed from the sixteen possible dimer blocks. Each coupling reaction was performed using almost stoichiometric amounts of each component in the presence of mesitylenesulfonyl tetrazole, as coupling reagent (S. Narang et al, U.S. Patent No. 4,059,592, 22 November 1977), for 30-45 min. at room temperature. After work-up, the desired product was isolated by the reversed-phased chromatographic technique developed by H.M. Hsiung et al, Nucleic Acid Res. <u>6</u>, 1371, 1979. After the completion of synthesis, the 5'-dimethoxytrityl group was removed using 2% benzenesulfonic solution in chloroform-methanol (J. Stawinski et al, Nucleic Acid Res. <u>4</u>, 353, 1977). All the other protecting groups were removed by two-step concentrated ammonia treatments (H.M. Hsiung et al, Nucleic Acid Res. <u>8</u>, 5753, 1980). Each of the unprotected oligomers was purified on PEI-tlc plate and sequenced by the mobility-shift method (C.D. Tu et al, Anal. Biochem. <u>74</u>, 73, 1976). Each fragment sequence was confirmed and their autoradiographs have been published (Nucleic Acid Res. <u>6</u>, 1371, 1979; <u>7</u>, 2199, 1979; <u>8</u>, 5753, 1980).

C. <u>Synthesis and Cloning of the Proinsulin Gene</u>

The human-like proinsulin gene which we prepared is given in formula 2. This codon sequence (base pairs 1 through 258) was selected by us and codes for the human-like proinsulin, the structure of which is shown in formula 1. The coding sequence (gene sequence) was based on that of the rat proinsulin gene (Ullrich et al, Science <u>196</u>, 1313, 1977) wherever the amino acids are in common. The amino acids in positions 3, 9, 30, 34, 37, 40, 42, 49, 52, 55, 60, 61, 62 and 69 for the human proinsulin are different than those in rat, thus in these positions a coding sequence for the human proinsulin was selected. In formula 2 the nucleotides 1-90, 91-195 and 196-258 code for human-like insulin chain B, chain C and chain A, respectively. The codons we selected form a unique combination.

The human-like proinsulin gene was assembled in the following way. The gene coding for the B-chain and a major portion of the C-chain was constructed as one unit which included the start signal (on fragments B-18 and B-19) and the coding sequence (nucleotides 1-178 of the upper strand and 1-182 of the lower strand of formula 2). This synthetic DNA was constructed from 32 synthetic fragments of 10 to 13 nucleotides in length. Four to six fragments were first joined together by cohesive-end ligation using DNA ligase to give six groups of fragments (a-f in formula 6). The sequence of each fragment was shown in formula 2. A similar scheme can be used for other proinsulin analogs.

Formula 6   A scheme for the construction of B+C chain of a
            human-like proinsulin gene.

a)   __B-18__   __B-1__   __B-2__

        __B-19__   __B-10__   __B-11__

b)   __B-3__   __B-4__   __B-5__

        __B-12__   __B-13__   __B-14__

c)   __B-6__   __B-7__

        __B-15__   __B-16__

d)   __B-8__   __C-1__   __C-2__

        __C-9__   __C-10__   __C-11__

e)   __C-3__   __C-4__

        __C-12__   __C-13__

Formula 6(cont.)

f)   C-5        C-6        C-7
    _____  _____  _____
        C-14       C-15       C-16

B-18

```
EcoRI
site                 start  1          178
5'  A A T T C C G G A T G ┌───────────────┐
3'        _____        │ B+C chain gene │ C T A G
             frag B-19    └───────────────┘ sau 3A
```

Structure I

In constructing each group of fragments, about 800 pmoles of each synthetic fragment (4 to 6 fragments) was phosphorylated at its 5'-end with $^{32}$P using polynucleotide kinase. The phosphorylated fragment was purified on a 20% polyacrylamide gel. About 500 pmoles of each purified fragment was annealed in 30 μl volume at 65°C and slowly cooled to 0°C. Concentrated DNA ligase buffer was added to give the following final concentrations: 50 mM Tris-HCl (pH 7.6), 10 mM $MgCl_2$, 10 mM dithiothreitol, 100 μM ATP. $T_4$ polynucleotide ligase (0.4 unit) was added and the mixture incubated at 12°C for 24-36 hours. The ligation product was purified on a 15% polyacrylamide gel (denaturing condition). The yield of fragments incorporated into each group (a through f) varied between 120 and 250 pmoles.

In the next step, one group was joined to the adjacent group (e.g. group a to b) by cohesive-end ligation until all six groups were connected to give structure I of formula 6. The ligation conditions are the same as given in the paragraph above. The yield of structure I was about 20 pmoles.

The cloned human-like insulin A-chain gene has been reported in a pending U.S. patent application No. 129,880, filed March 27, 1980, by S.A. Narang and R.J. Wu, which is hereby incorporated by reference. The insulin A-chain gene has been retrieved (recovered) from the clone by digestion with EcoRI restriction enzyme, which exposed the EcoRI site eight nucleotides away from the A-chain gene as shown in formula 7.

Formula 7   The Cloned Insulin A-chain Gene.

```
EcoRI
 site           start 196              258  stop
5' A A T T C A T G | A-chain gene,      | T G A G|            pBR322    - - -3'
                   |                    |        |
         G T A C  | base pairs 196-258 | A C T C C T A G|               - - -
                                                 Bam site
```

Note that the 3' end (right-hand end) of the cloned A-chain gene at this stage is still joined to the stop adaptor and pBR322 plasmid cloning vehicle. The eight extra nucleotides at the 5' end (lefthand side) of the insulin A-chain gene (which includes the start signal) has been removed by using a MboII trimming adaptor. The DNA in formula 7 was first repaired by using dATP and dTTP in the presence of AMV reverse transcriptase. A decanucleotide MboII adaptor for trimming DNA was next blunt-end ligated to the repaired end to give:

Formula 8   MboII Adaptor Ligated to the Repaired Cloned A-chain Gene

```
       MboII     start 196      258
5' TCTTCGAAGAAATTCATG |           | TGAG|        pBR 322   | - - - -
                      | A-chain gene |    |                |
3' AGAAGCTTCTTTAAGTAC |           | ACTCCTAG|              | - - - -

   MboII retrieving adaptor
```

The blunt-end ligation was carried out in 15 µl of a reaction mixture containing 50 mM Tris-HCl (pH 7.6), 10 mM $MgCl_2$, 10 mM dithiothreitol 100 µM ATP, 100 pmoles of the self-complementary MboII adaptor, 30 pmoles of cloned A-chain gene, and 0.3 units of $T_4$ polynucleotide kinase. Incubation was at 12°C for 40 hours.

-12-

Cleavage of DNA shown in formula 8 with MboII restriction enzyme (which cleaves DNA at the arrows, eight nucleotides away from recognition sequence GAAGA), followed by E. coli polymerase I (in the presence of $^{32}$P dCTP or cold dCTP to insure that the end of DNA is flushed), results in the removal of the eight extra base pairs from the A-chain gene. The retrieved A-chain gene was next cleaved with BamHI restriction enzyme, which gave the following structure:

Formula 9   Retrieved Insulin A-chain Gene.

```
         196        258    stop
        ┌──────────────┐   T  G  A  G
        │ A-chain gene │
        │              │   A C T C C T A G
        └──────────────┘       BamHI site
```

The retrieved insulin A-chain gene was blunt-end ligated to segments C-8 and C-17 to give structure II.

```
        C-8
  SAu3A                196        258    stop
  GATCACTTCAAAAGCGT┌──────────────┐      TGAG
                   │ A-chain gene │
                   │              │      ACTCCTAG
        ──────────┘└──────────────┘          BamHI site
        C-17
```

Structure II

Structure II was then joined to structure I at their Sau3A sites by cohesive-end ligation under the same conditions as given above for each group of fragments, to give the proinsulin (B+C+A chain) gene as shown in formula 10, lower part. This same synthesis strategy can be adapted to prepare other proinsulin gene analogs.

Formula 10   Scheme for the construction of a human-like
proinsulin gene from synthetic fragments

B-18                                         C-8
EcoRI
site start   1              178      Sau3A    196        258 stop
AATTCCGGATG  ┌─────────────┐         GATC    ┌──────────┐ TGAG
             │ B+C chain gene│ CTAG  +       │ A chain gene│ ACTCCTAG
frag B-19    └─────────────┘  Sau3A          C-17          BamHI
                                                           site
        Structure I                              Structure II

                            ligation

        EcoRI
        site start   1              258    stop
        AATTCCGGATG  ┌─────────────┐       TGAG
                     │              │
                     │ B+C+A chain gene│
                     └─────────────┘       ACTCCTAG
                                           BamHI site

                    Proinsulin Gene

It must be pointed out that the human-like proinsulin gene can
also be constructed by joining the same 32 fragments of the B+C chain and
the retrieved A-chain gene in several different ways.

The natural human proinsulin contains a C-chain consisting of
35 amino acids. The efficiency of removing the C-chain by peptidases to
produce biologically active insulin (B+A chains) may be influenced by the
length of the C-chain. Thus, we designed several methods to produce proinsulin analogs by changing the length of the C-chain gene, which would
lead to proinsulin analogs with different lengths of the C-chain. One or
several of the proinsulin analogs may prove to be superior than the
natural proinsulin in giving a higher yield of the biologically active
insulin, and possibly the modified C-chains will have less physiological
side effects.

(a) In one method, the length of the C-chain gene (normally 35 amino
acids X3 = 105 base pairs) can be assembled by omitting two or more fragments to give only 93, 84 or 72 base pairs, respectively. This can be
accomplished by constructing the B+C chain in a similar way as that in
formula 6 except that groups d, e, f have been redesigned (see formula
11) by eliminating fragments C-5 and C-13 to give a C-chain shorter by 12
base pairs (see formula 11). In a similar manner, the elimination of

-14-

fragments C-3, C-4, C-11, and C-12, gave a C-chain shorter by 21 base pairs, or the elimination of fragments C-3, C-4, C-5, C-11, C-12, and C-13, gave a C-chain shorter by 33 base pairs.

### Formula 11

Scheme for the construction of a shorter B+C chain of a human-like proinsulin gene. Groups a), b) and c) are the same as in formula 6 and are not shown.

d)

| B-8 | C-1 | C-2 |
|-----|-----|-----|

C-9      C-10 —GGGT

e)

CCCA— C-3      C-4

C-11      C-12 —GGGT

f)

CCCA— C-6      C-7

C-14      C-15      C-16

These constructions are possible since the nucleotide sequence at the junctions of the eliminated fragments (C-2, C-4, and C-5) are identical and they all contain a 3' protruding sequence d(G-G-G-T) in the upper strand (see formula 2, underlined nucleotides, and formula 11). Thus, we can eliminate one or two or three segments from the upper strand and still allow annealing with the d(C-C-C-A) sequence in the lower strand to give C-chains of different length. Once the different shortened C-chains are assembled, each will be joined to groups a), b), and c) of formula 6 to give shortened B+C chain gene similar to structure I of formula 10. Finally, shortened structure I is jointed to structure II of formula 10 to give shortened proinsulin genes as analogs of the normal gene.

(b)  The C-chain gene can be shortened to only 18 base pairs by ligating the following synthetic fragments between the right-hand end of the B-chain gene and the left-hand end of the A-chain gene as follows:

<u>Formula 12</u>    Mini-C-chain coding sequence of human proinsulin gene

```
                      Arg Arg Lys Leu Lys Arg
5'--- -┌──────────────CGT CGT┐┌AAG CTT AAG CGT┐──────────────────────
       │              ┆       ┆               ┆
       │              ┆       ┆               ┆
3'--- --- --- ---┘    GCA GCA TTC GAA TTC GCA┘──────────────────────
   B-chain                mini-C-chain gene        A-chain gene
   fragments
```

The human-like proinsulin gene (chain B-C-A), or its analogs, which carries a start signal at the 5' end (left-end) and the stop signal at the 3' end (right-end) was jointed to <u>EcoRI</u> and <u>BamHI</u>-digested pBR322 plasmid and the resulting hybrid DNA used to transform <u>E. coli</u> (strain 5346).  This strain and the containment facilities used conformed to current recombinant DNA regulations.  The desired clones were identified by colony hybridization (Grunstein and Hogness, Proc. Nat. Acad. Sci. <u>72</u>, 3961, 1975) with $^{32}$P-labelled C-chain fragment (17-32 nucleotides long), and $^{32}$P-labelled cloned A-chain fragment.  Among the positive clones, the exact sequence of several cloned proinsulin genes was determined by DNA sequence analysis.

The C-chain gene in the cloned human-like proinsulin gene also can be shortened after digesting the latter with <u>Sau3A</u> restriction enzyme to give a DNA structure in which the right-hand end is similar to that shown in Structure I above, and the left-hand end includes all of the B-chain gene and an additional 325 base pairs of the DNA from the cloning vehicle pBR322.  This molecule can be shortened from the right-hand end in two ways:

(i)  by removing the 4 protruding nucleotides (5' GATC) by S1 nuclease digestion (Vogt et al, Europ. J. Biochem. <u>33</u>, 192, 1973) and joining a <u>Mbo</u> II retrieving adaptor by blunt-end ligation.  After carrying out the steps shown in Formula 5 above, 8 base pairs can be removed from the right-hand end to shorten this part of the C-chain by 12 base pairs (4+8).  By repeating the <u>Mbo</u> II retrieving adaptor procedure (Formula 5) one or more cycles, but using the following adaptor (Formula 13), 6 base pairs can be removed in each cycle from the right-hand end of the already shortened C-chain gene.


Formula 13   A Mbo II retrieving adaptor containing two additional base pairs at each end (N, N' are any complementary nucleotides)

5' N N     T C T T C G A A G A     N'N'

3' N'N'    A G A A G C T T C T     N N


By using the above methods, the C-chain gene of the proinsulin can be shortened by a total of 12, 18, 24 or 30 base pairs leftward from the nucleotide #182 of Formula 2 above.

(ii)  by digesting the DNA with a combination of exonuclease III and S1 nuclease (Wu et al, Biochem. 15, 734, 1976; Roberts et al, Proc. Natl. Acad. Sci. 76, 760, 1978) or with BAL31 (Legerski et al, Nucleic Acids Res. 5; 1445, 1978; Talmadge et al, Proc. Natl. Acad. Sci. 77, 3369 and 3988, 1980). In this method from about 6 to 81 base pairs can be removed and those DNA molecules with a multiple of 3 base pairs removed (e.g. 6, 9, 12, 15, .... 81) can be selected after cloning and DNA sequence analysis.

The C-chain gene can be lengthened by digesting the cloned human-like proinsulin gene with Sau3A restriction enzyme as described above and inserting DNA fragments of different lengths. For example, the 5' protruding end (5' GCTA) can be joined by cohesive-end ligation to another molecule of synthetic fragment C-16 followed by C-7. After repair synthesis with 4 dNTP and the AMV reverse transcriptase, the blunt-ended DNA is now 13 base pairs longer. When this molecule is blunt-end ligated to the S1 nuclease digested structure II above, which digestion removed 4 base pairs from the retrieved and modified insulin A-chain gene, the net result is an addition of 9 base pairs to the C-chain of the proinsulin gene (codes for three additional amino acids). Using the same procedure but adding C-16, C-7, C-15 and C-6 fragments, the end result is the addition of 21 base pairs (seven additional amino acids). Similarly, other multiples of three base pairs can be added.

After the modification of the C-chain gene, which is still attached to the B-chain gene and some pBR322 DNA, it can be blunt-end ligated to the retrieved and modified insulin A-chain gene (Structure II, above) after cleaving off the 4 protruding nucleotides (5' GATC at the Sau3A site) at the left-hand end by S1 nuclease (as mentioned in the previous paragraph). The product is then digested by EcoRI and BamHI restriction enzymes to release the shortened proinsulin gene (corresponding to the parent structure shown at the bottom of Formula 10, above), and ligated by cohesive-end ligation to EcoRI- and BamHI-digested pBR322 plasmid and cloned as described previously.

For maximum expression, the cloned proinsulin gene can be excised by digestion of the cloned hybrid plasmid DNA with EcoRI and BamHI endonucleases. The BamHI end can be converted to an EcoRI end by using an EcoRI-BamHI conversion adaptor of the type reported in U.S. Patent Application No. 129,880, filed March 27, 1980 by S.A. Narang and R.J. Wu. The conversion adaptor has the structure:

Formula 14   EcoRI-BamHI Conversion Adaptor

```
     EcoRI
     site
5'   A A T T C T A A C A G T C G

3'           G A T T G T C A G C C T A G
                                 BamHI site
```

The resulting adapted proinsulin gene carrying an EcoRI site at each end can be ligated to a plasmid such as pBGP 120 (Polisky et al, Proc. Nat. Acad. Sci. 73, 3900, 1976) or pLL70 (Lau and Wu, unpublished result), adjacent to a strong lactose promoter for expression. This reconstructed hybrid plasmid DNA carrying the proinsulin gene can be used to transform E. coli cells and to direct the synthesis of large amounts of the human proinsulin, which is a natural precursor of biologically active insulin. Alternatively, the cloned proinsulin gene can be joined to the lactose promoter and then to plasmid pKN402 (Uhlin et al, Gene 6, 91, 1979), or a derivative of it, which can produce over 200 copies of the plasmid per E. coli cell. The copy number of this plasmid is at least seven times more than that of pBGP120 or pBR322. Thus, over 200 copies of the proinsulin gene can be synthesized per E. coli cell to yield up to 500,000 copies of the proinsulin protein per cell.

The E. coli proinsulin clone is being maintained in the Dept. of Biochemistry at Cornell University, Ithaca, N.Y. and will be made available as required.

/
0068701

CLAIMS:

1.      An insulin C-chain gene analog shortened or lengthened from the normal gene length by the deletion or addition of nucleotides, respectively.

2.      The insulin C-chain gene analog of claim 1 shortened or lengthened by the deletion or addition of nucleotides in multiples of three.

3.      The insulin C-chain gene of claim 1 wherein the normal gene length is the duplex DNA nucleotides 91-195 of formula 2 herein.

4.      An insulin C-chain partial gene as in claim 3 having the duplex DNA sequence C-1 to C-7 plus C-9 to C-16 of formula 2, suitable for ligation to appropriately adapted insulin A-chain and B-chain genes.

5.      The insulin C-chain gene of claim 3 shortened 12 base pairs by deletion of fragments C-5 and C-13.

6.      The insulin C-chain gene of claim 3 shortened 21 base pairs by deletion of fragments C-3, C-4, C-11 and C-12.

7.      The insulin C-chain gene of claim 3 shortened 33 base pairs by deletion of fragments C-3, C-4, C-5, C-11, C-12 and C-13.

8.      The insulin C-chain gene of claim 3 shortened by one of 12, 18, 24, 30, or more base pairs leftward from nucleotide 182 of Formula 2.

9.      The insulin C-chain gene of claim 3 shortened by one of 6, 9, 12, 15, .... up to 81 base pairs, in multiples of 3, leftward from nucleotide 182 of Formula 2.

10.     The insulin C-chain gene of claim 3 lengthened by multiples of three  base pairs by ligating DNA fragments of selected lengths at the Sau3A site (5' GATC) of the C-chain gene.

CLAIMS (cont.)

11.     The insulin C-chain gene analog having the formula:
        CGT CGT AAG CTT AAG CGT
        GCA GCA TTC GAA TTC GCA

12.     An insulin C-chain gene including inserted nucleotides selected
to form at least one restriction enzyme recognition site.

13.     Proinsulin gene analogs in which the normal C-chain DNA has
been shortened by deletion of nucleotides or lengthened by addition of
nucleotides in multiples of three.

14.     The proinsulin gene analogs of claim 13 ligated to a replicable
cloning vehicle.

15.     A method of assembling a modified proinsulin gene comprising:
(a)  providing a combined insulin B + partial C chain gene analog having
the enzyme recognition site for Sau3A (3' CTAG 5') in the lower DNA
strand at the free partial C-chain end,
(b)  providing an insulin A-chain gene carrying the other part of the
C-chain gene analog and having the complementary recognition site for
said enzyme 5' GATC 3' at the upstream end in the upper DNA strand of the
remainder of the C-chain gene analog, and
(c)  ligating the two genes (a) and (b) at their Sau3A sites to form the
complete proinsulin gene.